(19) Europäisches Patentamt
European Patent Office
Office européen des brevets

(11) **EP 3 037 413 A1**

(12) **EUROPEAN PATENT APPLICATION**

(43) Date of publication:
**29.06.2016 Bulletin 2016/26**

(51) Int Cl.:
***C07D 209/86*** *(2006.01)* ***H01L 51/56*** *(2006.01)*

(21) Application number: **14199899.7**

(22) Date of filing: **22.12.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Solvay SA**
**1120 Bruxelles (BE)**

(72) Inventor: **Virboul, Morgane**
**1060 Brussels (BE)**

(74) Representative: **Dr. Langfinger & Partner**
**In der Halde 24**
**67480 Edenkoben (DE)**

(54) **Process for the manufacture of dibenzosuberane compounds**

(57) A process for the manufacture of dibenzosuberane compounds of formula (1)

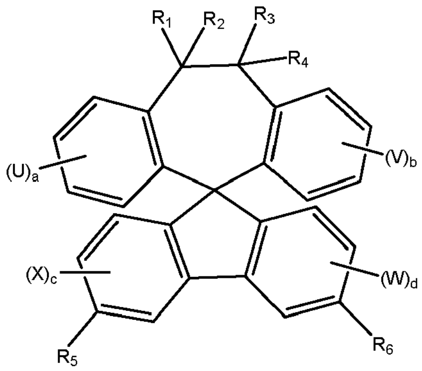

(1)

wherein

$R_1$ to $R_4$, which may be the same or different, are selected from H or halogen, cyano, hydroxyl, alkyl, alkenyl, alkynyl, heteroalkyl, heteroalkenyl, heteroalkynyl and alkoxyl with 1 to 18 carbon atoms or $R_1$ and one of $R_3$ and $R_4$ form a chemical bond,

U, V, W, X , which may be the same or different at each occurrence, are halogen, cyano, hydroxyl, alkoxy, alkyl, alkenyl, alkynyl, heteroalkyl, heteroalkenyl, heteroalkynyl, aryl, heterocyclyl, and heteroaryl group,

$R_5$ and $R_6$, which may be the same or different at each occurrence, are hydrogen, halogen, cyano, hydroxyl, alkoxy, alkyl, alkenyl, alkynyl, heteroalkyl, heteroalkenyl, heteroalkynyl, aryl, heterocyclyl, and heteroaryl, provided that at least one of $R_5$ and $R_6$ is not hydrogen, and

a and b, which may be the same or different, are 0,1,2 or 3 and c and d, which may be the same or different, are 0, 1 or 2.

EP 3 037 413 A1

## Description

The present invention relates to a process for the manufacture of dibenzosuberane compounds and to novel dibenzosuberane compounds as such.

Organic light-emitting diodes (OLEDs) are an important feature in modern display and lighting technologies, such as, for example, full-color flat displays, flexible displays, and solid-state lighting. Phosphorescent organic light-emitting diodes (PhOLEDs), an important class of OLEDs, are theoretically capable of achieving a 100% internal quantum efficiency by fully harvesting both singlet and triplet excitons. Therefore, PhOLEDs have attracted much attention for their applications in full-color displays and lighting. One promising strategy to obtain highly efficient PhOLEDs is to utilize high triplet energy materials to confine triplet excitons inside an emission layer (EML) in multilayered device structures.

High triplet energy materials are mainly used in EMLs as a host material or in adjacent hole transport layers (HTL) and electron transport layers (ETL). Use of high triplet energy confines triplet excitons inside the EML and suppresses triplet exciton quenching. In multilayered PhOLEDs, the ETL plays an important role in facilitating electron-injection/transport from a cathode while also acting as efficient exciton blocker. It is therefore preferable that the ETL have good electron-transport property, wide energy gap and high triplet energy. A highest occupied molecular orbital (HOMO) level of the electron-transport material is preferably deep enough to block hole carrier leakage and a lowest unoccupied molecular orbital (LUMO) level is preferably low enough to enable efficient electron injection from the cathode. Electron transport materials with high triplet energy preferably exhibit electrochemical, photochemical, and morphological stability.

Various electron-transport materials (ETMs) such as pyridine, phenylpyrimidine, triazine, quinoline, and phosphine oxide (PO) derivatives have been mainly used to achieve high-performance PhOLEDs. Dibenzothiophene-S,S-dioxide and thiophene-S,S-dioxide oligomers and polymers have not been usually viewed as suitable ETMs for PhOLED devices. Although they function as good ETMs for devices with high electron mobilities ($10^{-4}$ - $10^{-3}$ cm2 $V^{-1}$ $s^{-1}$), their low band gap and low triplet energy are in many cases not suitable for efficient PhOLEDs, especially for a blue triplet emitter with high triplet energy.

Korean Patent Application KR2012047038 describes amine derivatives and organoelectroluminiscent devices employing these compounds. There are a number of compounds disclosed comprising a dibenzosuberane unit coupled to a fluorene unit thereby yielding the following core structure

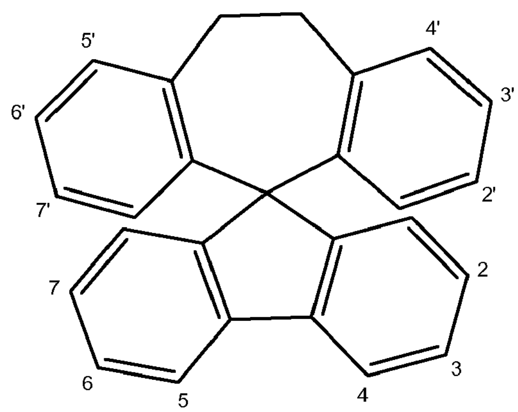

which may be substituted by an amine compound which amine compound comprises a structural element with at least three fused rings with a central ring comprising a nitrogen atom through which the amine is attached to the dibenzosuberane core. One of the non-central fused rings is a non aromatic carbocyclic ring. The general basic structure given for the amine substituent is

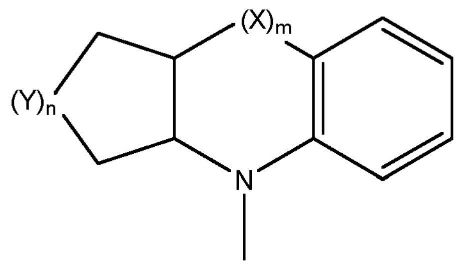

and the attachment to the dibenzosuberane core is through the nitrogen atom.

Japanese Patent Application JP2010/024149 discloses compounds having a core element as in KR2012047038 and attached thereto substituents comprising one or more aromatic or non aromatic rings. The attachment to the core is through a carbon atom of the substituent.

All the dibenzosuberane compounds with the core structure shown above described in the prior art are substituted in one or more of positions 2, 2', 7 and 7' but there are no compounds disclosed bearing substituents in one or more of positions 3 and 6. Compounds with such a substitution, however, could have an interesting property spectrum which property spectrum is not easily expected for compounds merely substituted in positions 2, 2', 7 and 7'.

Substitution in 3 and/or 6 positions cannot be obtained through direct halogenation of the dibenzosuberane core (as substitution in positions 2, 2', 7 and 7' is) and accordingly there existed a need for a suitable process to obtain dibenzosuberane compounds having a core as shown above with substitution in 3 and/or 6 positions.

It was thus an object of the present invention to provide a process for the manufacture of dibenzosuberane compounds of formula (1)

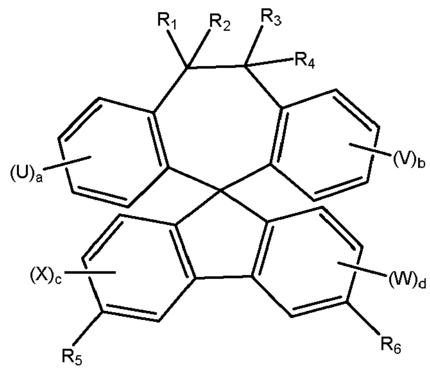

(1)

wherein

$R_1$ to $R_4$, which may be the same or different, are selected from H or halogen, cyano, hydroxyl, alkyl, alkenyl, alkynyl, heteroalkyl, heteroalkenyl, heteroalkynyl and alkoxyl with 1 to 18 carbon atoms or $R_1$ and one of $R_3$ and $R_4$ form a chemical bond,

U, V, W, X , which may be the same or different at each occurrence, are halogen, cyano, hydroxyl, alkoxy, alkyl, alkenyl, alkynyl, heteroalkyl, heteroalkenyl, heteroalkynyl, aryl, heterocyclyl, and heteroaryl group, $R_5$ and $R_6$, which may be the same or different at each occurrence, are hydrogen, halogen, cyano, hydroxyl, alkoxy, alkyl, alkenyl, alkynyl, heteroalkyl, heteroalkenyl, heteroalkynyl, aryl, heterocyclyl, and heteroaryl, provided that at least one of $R_5$ and $R_6$ is not hydrogen, and a and b, which may be the same or different, are 0,1,2 or 3 and c and d, which may be the same or different, are 0, 1 or 2.

This object has been achieved withh the process in accordance with claim 1.

Preferred embodiments of the process of the invention are described in the dependent process claims and the detailed description hereinafter.

Another embodiment of the present invention are compounds of formula (8)

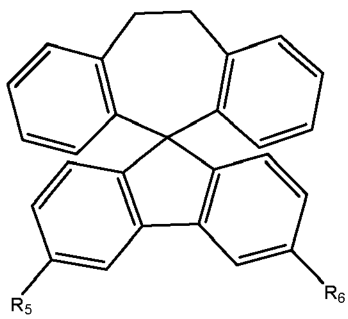

(8)

wherein $R_5$ and $R_6$ have the meaning described above.

As used herein, the term "halogen" means a halide radical selected from the group consisting of fluoride,

chloride, bromide and iodide.

As used herein, the term "alkyl" means a monovalent straight, branched or cyclic saturated hydrocarbon radical, more typically, a monovalent straight or branched saturated ($C_1$-$C_{40}$)hydrocarbon radical, such as, for example, methyl, ethyl, n-propyl, isopropyl, n-butyl, isobutyl, tert-butyl, hexyl, octyl, hexadecyl, octadecyl, eicosyl, behenyl, tricontyl, and tetracontyl.

As used herein, the term "alkoxy" means an alkyl group bonded to an oxygen atom, i.e. a group OR where R denotes an alkyl group as herein defined.

As used herein, the term "cycloalkyl" means a saturated cyclic $C_5$-$C_{40}$-hydrocarbon radical, more typically a saturated $C_5$-$C_{22}$-hydrocarbon radical, that includes one or more cyclic alkyl rings, which may optionally be substituted on one or more carbon atoms of the ring with one or two $C_1$-$C_6$-alkyl groups per carbon atom, such as, for example, cyclopentyl, cycloheptyl and cyclooctyl.

As used herein, the term "alkenyl" means an unsaturated straight or branched $C_2$-$C_{40}$-hydrocarbon radical, more typically an unsaturated straight, branched, $C_2$-$C_{22}$-hydrocarbon radical, that contains one or more carbon-carbon double bonds, including, for example, ethenyl (vinyl), n-propenyl, and iso-propenyl, and allyl.

As used herein, the term "alkynyl" means an unsaturated straight or branched ($C_2$-$C_{40}$) hydrocarbon radical, more typically an unsaturated straight, branched, $C_2$-$C_{22}$-hydrocarbon radical, that contains one or more carbon-carbon triple bonds, including, for example, ethynyl, propynyl, and butynyl.

The term "heteroalkyl" means an alkyl group wherein one or more of the carbon atoms within the alkyl group has been replaced by a hetero atom, such as, for example, nitrogen, oxygen, or sulfur.

The term "heteroalkenyl" means an alkenyl group wherein one or more of the carbon atoms within the alkenyl group has been replaced by a hetero atom, such as, for example, nitrogen, oxygen, or sulfur.

The term "heteroalkynyl" means an alkynyl group wherein one or more of the carbon atoms within the alkynyl group has been replaced by a hetero atom, such as, for example, nitrogen, oxygen, or sulfur.

As used herein, the term "aryl" means a monovalent unsaturated hydrocarbon radical containing one or more carbon rings having a total number of $\pi$-electrons following the so called Hückel rule, i.e. having 4 n+2 $\pi$-electrons with n being an integer of at least 1. Aryl radicals include monocyclic aryl and polycyclic aryl. "Polycyclic aryl" refers to a monovalent unsaturated hydrocarbon radical containing more than one six-membered carbon ring in which the unsaturation may be represented by three conjugated double bonds wherein adjacent rings may be linked to each other by one or more bonds or divalent bridging groups or may be fused together. Aryl radicals may be substituted at one or more carbons of the ring or rings with any substituent described herein. Examples of aryl radicals include, but are not limited to, phenyl, methylphenyl, isopropylphenyl, tert-butylphenyl, methoxyphenyl, dimethylphenyl, trimethylphenyl, chlorophenyl, trichloromethylphenyl, triisobutyl phenyl, anthracenyl, naphthyl, phenanthrenyl, fluorenyl, and pyrenyl.

As used herein, the term "heterocycle" or "heterocyclic" refers to compounds having a saturated or partially unsaturated cyclic ring structure that includes one or more hetero atoms in the ring. The term "heterocyclyl" refers to a monovalent group having a saturated or partially unsaturated cyclic ring structure that includes one or more hetero atoms in the ring. Examples of heterocyclyl groups include, but are not limited to, morpholinyl, piperadinyl, piperazinyl, pyrrolinyl, pyrazolyl, and pyrrolidinyl.

As used herein, the term "heteroaryl" means a monovalent group having at least one aromatic ring that includes at least one hetero atom in the ring, which may be substituted at one or more atoms of the ring with hydroxyl, alkyl, alkoxyl, alkenyl, halogen, haloalkyl, monocyclic aryl, or amino. Examples of heteroaryl groups include, but are not limited to, thienyl, pyrrolyl, pyridinyl, pyrimidinyl, pyrazinyl, triazinyl, pyridazinyl, tetrazolyl, and imidazolyl groups. The term "polycyclic heteroaryl" refers to a monovalent group having more than one aromatic ring, at least one of which includes at least one hetero atom in the ring, wherein adjacent rings may be linked to each other by one or more bonds or divalent bridging groups or may be fused together. Examples of polycyclic heteroaryl groups include, but are not limited to, indolyl and quinolinyl groups.

The process in accordance with the present invention comprises four subsequent reaction steps.

In the first step a) a substituted aryl halide of formula (2) is reacted with a boronic acid derivative of formula (3) at a temperature in the range of from 60 to 200 °C, preferably of from 70 to 180°C

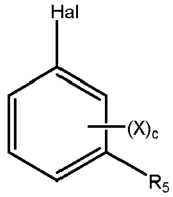
(2)

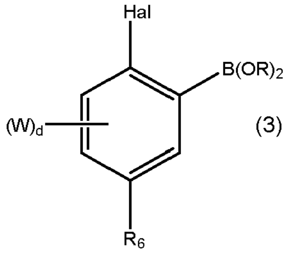
(3)

Hal is a halogen atom selected from the group consisting of fluorine, chlorine, bromine and iodine, preferably chlorine, bromine and iodine, particularly preferred bromine.

R is hydrogen or a $C_1$-$C_8$-hydrocarbyl group suitable for the coupling reaction. Preferred hydrocarbyl groups are alkyl and alkoxy groups, most preferred R is hydrogen.

Compounds of formula (2) and (3) have been widely described in the literature and processes for their manufacture are known to the skilled person. A significant number of compounds of this type is also commercially available.

Step a) is carried out in the presence of a transition metal catalyst and a base. Step a) is principally known to the skilled person as Suzuki or Suzuki-Miyaura reaction or Suzuki coupling. This name is generally used for the reaction of an aryl boronic acid derivative with an aryl halide and such reactions have been described as such in the literature.

The transition metal catalysts used in the reaction which have been most widely described are Pd(0) catalysts which e.g. may be obtained by combining a palladium salt such as palladium acetate or bis(dibenzylideneacetone)palladium (generally referred to as $P(dba)_2$) with a phosphine ligand such as triphenylphosphine or tri-tert. butylphosphine, to name only two examples.

The amount of catalyst, based on the amount of the starting materials is usually in the range of from 0.1 to 20 mol%, preferably in the range of from 0.5 to 10 mol%.

Ni-catalysts are also suitable for reactions of this type, albeit generally higher amounts of catalyst are most often necessary than for Pd catalysts. Since Ni is not as expensive as Pd, however, the overall economic situation for Ni catalysts is still often at least comparable to Pd catalysts.

The skilled person will select the best suitable catalyst based on his professional knowledge and the specific target compound. Overviews concerning best suited transition metal catalysts are given in Chem. Rev. 95(7), 2457-2483 and in Chem. Soc. Rev. 42(12), 5270-5298 to which reference is made here for further details.

The Suzuki coupling reaction of step a) of the process of the present invention may be carried out in a suitable solvent capable of dissolving the raw materials. The skilled person will select the best suited solvent based on his professional experience so that no further details are necessary here. Just by way of example, toluene, xylene, tetrahydrofuran, dioxane, tetralin, quinolone, nitrobenzene, dimethyl sulfoxide and N,N-dimethylformamide may be mentioned.

Step a) requires the use of a base and suitable bases are also known to the skilled person and have been described in the literature. Often used are potassium carbonate, sodium carbonate, cesium carbonate, sodium hydroxide, sodium tert-butoxide, potassium tert-butoxide as examples for inorganic bases and pyridine, triethylamine and picoline as organic bases.

The molar ratio of aryl halide to boronic acid derivative is usually in the range of from 0.5:1 to 1.5: 1 and preferably in the range of from 0.8:1 to 1.2:1, most preferably the two reactants are used in approximately equimolar amounts.

A wide variety of biphenyl compounds of formula (4)

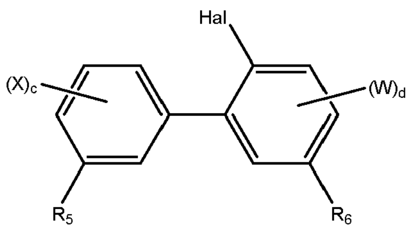

(4)

can be obtained in this way. The halogenated biphenyl compound obtained after step a) has the two substituents $R_5$ and $R_6$ which form the substituents in positions 3 and 6 of the final product. Thus, the compound of formula (4) is a key intermediate in the process of the present invention as it determines the positions of $R_5$ and $R_6$ in the desired position in the final product.

A wide variety of compounds with vastly different substituents $R_5$ and $R_6$ are available through step a) of the process in accordance with the present invention which may be used in subsequent step b).

One group of compounds, which is of particular interest due to their property spectrum are compounds wherein one of $R_5$ and $R_6$ is hydrogen.

Below a few compounds of formula (4) which belong to this group, the final products of can be expected to show an interesting property spectrum, are given:

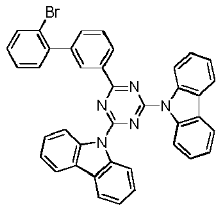 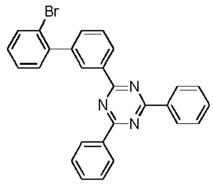 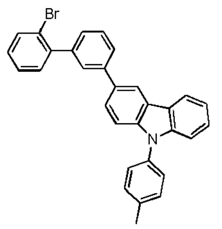 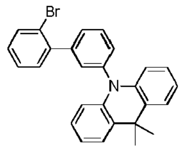

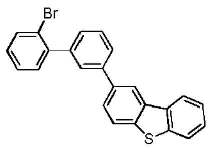 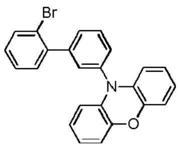 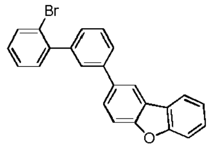 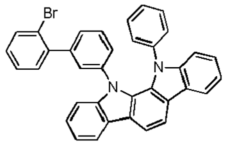 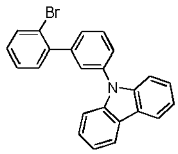

However, as mentioned, a vast variety of compounds of formula (4) with a great variation in substituents $R_5$ and $R_6$ may be obtained.

Step b) of the process in accordance with the present invention is a lithium-halogen exchange reaction which is known as such to the skilled person and has been described in the literature.

The skilled person will choose suitable lithium compounds for the exchange reaction b) based on his professional experience and thus no further details need to be given here. Just by way of example alkyl lithium compounds may be mentioned as a preferred group of lithium compounds. Tert-butyl lithium, sec-butyl lithium and n-butyl lithium are amongst the most easily available compounds of this group and thus are preferably used. Other alkyl lithium compounds with other alkyl groups are also suitable however, but often economically less preferred compared to the butyl lithium compounds.

The reaction of step b) is preferably carried out in a suitable solvent not decomposing the alkyl lithium compound. Tetrahydrofuran, diethyl ether and alkanes such as n-hexane may be mentioned here by way of example.

The reaction of step b) is carried out at a temperature in the range of from -100 to 30°C, preferably in the range of from -80 to 25°C.

The reaction time depends on the reaction temperature used but is in many cases in the range of from 3 h to 48 h, preferably of from 6 h to 24 h.

The reaction product of step b) is lithiated biphenyl compound (5).

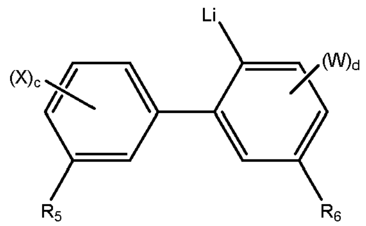

(5)

wherein the substituents have the meaning as described above.

The compound of formula (4) and the lithiating species, preferably an alkyl lithium compound are generally used in a molar ratio of 0.7 to 1 to 1.3 to 1, preferably of from 0.85:1 to 1.2:1 and even more preferably in approximately equimolar amounts. A slight excess of the alkyl lithium compound has shown to be advantageous in some cases.

In step c) of the process in accordance with the present invention, lithiated compound (5) is reacted with a dibenzosuberone compound (6)

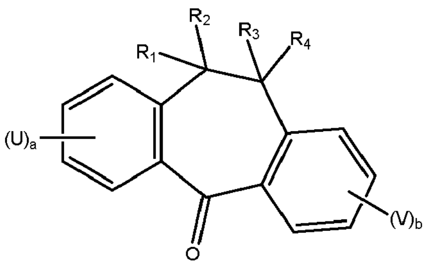

(6)

wherein R1 to R4 and U and V have the meaning as defined in claim 1, to obtain the intermediate alcohol compound of formula (7):

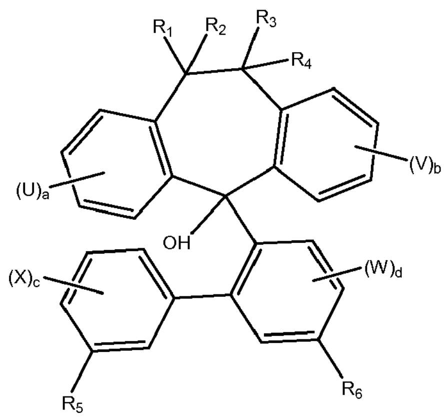

(7)

wherein the substituents have the meaning as defined in claim 1 and described hereinbefore.

In preferred dibenzosuberone compounds of formula (6) a and b are 0, i.e. the phenyl rings bear no substituents.

$R_1$ to $R_4$, which may be the same or different at each occurrence are preferably hydrogen or alkyl groups with 1 to 18 carbon atoms. In other preferred embodiments $R_1$ and one of $R_3$ and $R_4$ form a chemical bond. Most preferred $R_1$ to $R_4$ are hydrogen or $R_1$ and $R_3$ or $R_4$ form a chemical bond and the remaining substituents $R_2$ and $R_3$ respectively $R_4$ are hydrogen.

Step c) of the process of the present invention is usually carried out in a suitable solvent dissolving the reactants and the skilled person will select the appropriate solvent based on his professional knowledge, the reactants and the target compound to be synthesized. The solvent as such is not particularly critical but tetrahydrofuran, diethyl ether and hexane have proved to be advantageous in a number of cases.

After completion of step c) the compound of formula (7) can be isolated by adding water to the reaction mixture to separate an aqueous and an organic layer, washing the organic layer after concentration with a low boiling solvent, such as ethyl acetate or dichloromethane and drying the organic layer under reduced pressure to obtain the compound of formula (7).

Whereas the work-up as described above is not mandatory after step c), it has proved to be advantageous in a number of cases to isolate and purify the compound of formula (7) before carrying out step d) of the process of the present invention.

The yield of the compound of formula (7) in many cases is at least 40 %, often at least 50% or at least 60 %, based on the starting material.

The final step d) of the process of the present invention is an acid mediated cyclisation of the compound of formula (7) to obtain the target compound of formula (1). This step is preferably carried out in the presence of catalytic amounts of a strong acid. The choice of strong acid is not critical and the skilled person is aware of suitable acids for cyclisation reactions of this type. By way of example trifluoromethane sulfonic acid and trifluoroacetic acid may be mentioned as organic acids and nitric acid as a representative of a strong inorganic acid. In principle, the selection of the strong acid is not critical and inorganic acids as well as organic acids are suitable and may be used.

The amount of acid used, relative to the amount of alcohol, is usually in the range of from 0.1 to 10 mol%, preferably in the range of from 0.5 to 8 mol%.

Step d) can be carried out in an appropriate solvent capable of dissolving starting compound (7). Halogenated hydrocarbons, e.g. dichloromethane may be mentioned here by way of example. The skilled person will select the appropriate solvent based on the alcohol compound used as starting material by using his professional experience.

Step d) is usually carried out at a temperature in the range of from -20 to 60°C and preferably in the range of from 0°C to 30°C, depending on the solvent chosen.

The reaction time is usually in the range of from 15 minutes to 12 hours, preferably in the range of from 0.5 h to 6 hours and in many cases reaction times of about 1 to 4 h are sufficient.

After the completion of step d) the reaction product may be worked up and purified by quenching the reaction medium with water and washing the organic phase with an appropriate aqueous solution of a salt (e.g. sodium carbonate) until the water phase has reached a pH of about 7. Thereafter the organic phase can be washed with water, dried with e.g. magnesium sulfate and concentrated in vacuo. Purification of the residue by flash chromatography can follow.

As a result the desired compound of formula (1) can be usually obtained in good yields exceeding 50 %, often exceeding 60 % and sometimes exceeding 80 %, based on starting material 7.

The process of the present invention provides an easy way to dibenzosuberane compounds of formula (1) with a substitution pattern having a substitution in positions 3 and/or 6 which were not easily accessible by the processes described in the prior art. In particular the process of the present invention opens the way to asymmetrically substituted compounds of formula (1) having a substitution other than hydrogen in only one of positions 3 and/or 6, which may be expected to show an interesting property spectrum for use in organic electronic devices.

Another embodiment of the present invention relates to novel compounds of formula (8) which can be obtained with the process of the present invention.

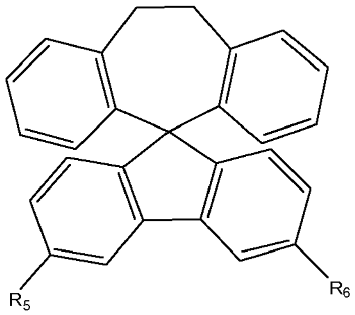

(8)

wherein $R_5$ and $R_6$, which may be the same or different at each occurrence, are hydrogen, halogen, cyano, hydroxyl, alkoxy, alkyl, alkenyl, alkynyl, heteroalkyl, heteroalkenyl, heteroalkynyl, aryl, heterocyclyl, and heteroaryl, provided that at least one of $R_5$ and $R_6$ is not hydrogen.

Preferred compounds of formula (8) having an asymmetric substition pattern as described above are the following:

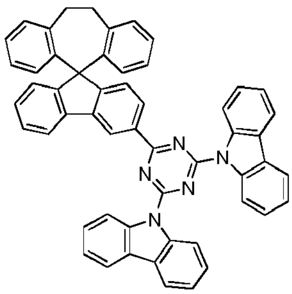

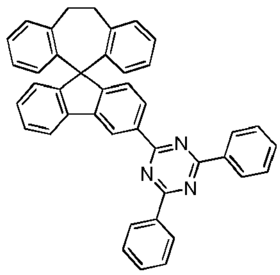

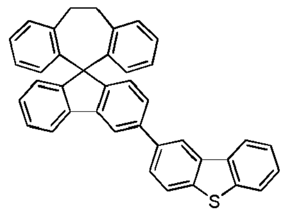

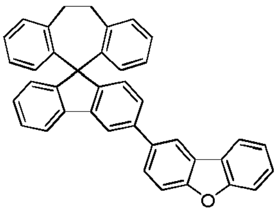

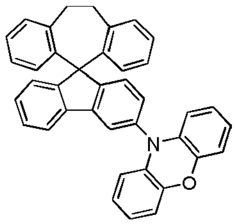

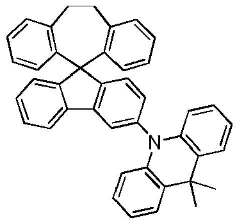

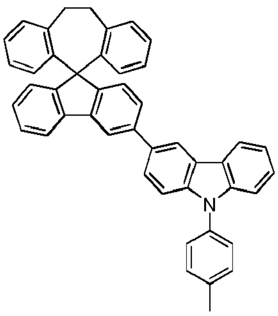

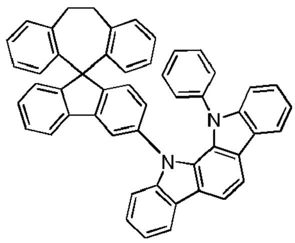

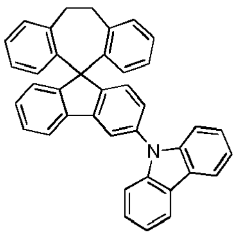

The following examples further describe the present invention.

Example 1 -

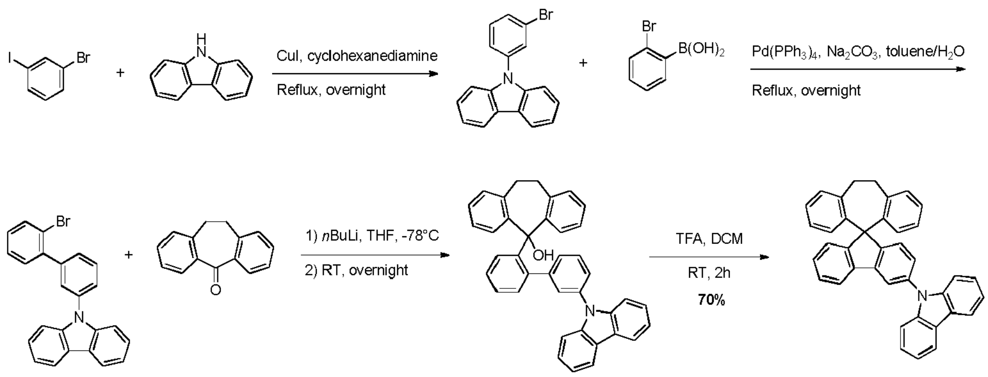


I II III

Compound **III** was obtained from compound **I**, which was synthesized as previously described in literature (Su, S-J., Cai, C., Kido, J. Chem. Mater. 2011, 23, 274-284; b) Lee, C. W., Im, Y., Seo, J-A., Lee, J. Y. Chem. Commun. 2013, 49, 9860-9862; c) Lee, C. W., Lee, J. Y. Dyes Pigments 2014, 101, 150-155) in a two-step procedure. Treatment of compound **I** with a stoichiometric quantity of nBuLi in THF led to the formation of the corresponding lithiated species which was further reacted with dibenzosuberone to yield the alcohol **II**. The condensation of the alcohol **II** in presence of a catalytic amount of trifluoroacetic acid in dichlormethane at room temperature yielded the expected product **III** in 70% overall yield.

Compound I (5.01 g, 12.6 mmol) was dissolved in THF (80 mL) and the resulting mixture was cooled down to -78°C. *n*BuLi 1.6M in hexane (9.5 mL, 15.2 mmol) was subsequently added to the solution and left to react for 2h at this temperature. A solution of dibenzosuberone (2.09 g, 10 mmol) in THF (50 mL) was slowly added and the temperature raised to room temperature. After 4h, the reaction mixture was quenched with water and the solvents evaporated. The residue was extracted with dichloromethane (3 x 20 mL) and washed with three times with water (3 x 20 mL). The organic phase was dried with $MgSO_4$ and concentrated *in vacuo.* The product was washed several times with hexane, dried and used directly without further purification in the following step.

Compound **II** was dissolved in dichloromethane (30 mL) and trifluoroacetic acid (7.5 mL, 44 mmol) was added dropwise. After 2h at room temperature, the resulting solution was quenched with water and the organic phase washed with a 1 M solution of $Na_2CO_3$ until the water phase had reached pH=7. The organic phase was then washed with water, dried with $MgSO_4$ and concentrated *in vacuo.* The residue was purified by flash chromatography using a mixture of hexane/ethyl acetate as the eluent to yield the pure product as a white solid (4.2 g, 82% yield).

$^1$H NMR (500 MHz, THF-$d_8$) $\delta$ = 8.29 (d, 2H, $J_{HH}$ = 7.5 Hz; Ar*H*), 8.08 (s, 1H; Ar*H*), 7.88 (d, 1H, $J_{HH}$ = 8 Hz; Ar*H*), 7.62-7.53 (m, 4H; Ar*H*), 7.47-7.41 (m, 5H; Ar*H*), 7.36-7.28 (m, 4H; Ar*H*), 7.18 (t, 2H, $J_{HH}$ = 7 Hz; Ar*H*), 6.99 (t, 2H, $J_{HH}$ = 7.5 Hz; Ar*H*), 6.64 (d, 2H, $J_{HH}$ = 8.5 Hz; Ar*H*), 3.55 (s, 4H; C$H_2$)

The molecule was fully characterized to determine its optical and electronic properties and the data are presented in the table below. The new compound exhibits a high triplet energy and a comparatively shallow HOMO level

Table 1:

| $E_T$ eV | $E_{optG}$ ev | HOMO eV | LUMO eV | $T_{decomp}$ | MW g/mol |
|---|---|---|---|---|---|
| 2.84 | 3.47 | -5.66 | -2.19 | 1%@313°C | 509.64 |

The triplet energy was calculated from the highest energy phosphorescence peak measured in 2-Me-THF at 77 K. (The photoluminescence measurements were performed on highly diluted (= $10^{-5}$ mol/L) solutions in spectroscopic grade of 2-methyl-THF using a HORIBA JOBIN YVON Fluoromax-4 P spectrofluorimeter. Measurements at 77 K were carried out using the FL-2013 Dewar liquid nitrogen assembly from HORIBA JOBIN YVON.

EoptG (ev) was measured from the onset wavelength determined by adsorption spectroscopy.

The HOMO and LUMO levels were determined from cyclic voltammetry measurements in solution as follows:

The measurements are performed at room temperature, under inert atmosphere, with a conventional three-electrode configuration, the solution being outgassed before use with a stream of argon for 5 - 10 min. The three-electrode cell may consist e.g. of a glassy carbon disk as working electrode, a Pt wire or a Pt rod as a counter electrode and a Pt wire or a carbon rod as pseudo-reference electrode. Ferrocene is used as an internal reference. Other cell configurations may also be used. The solvents used for the determination of the HOMO and LUMO levels are respectively anhydrous dichloromethane and anhydrous tetrahydrofuran, the supporting electrolyte is 0.1 M tetrabutylammonium hexafluorophosphate and the host concentrations are 2 - 0.5 millimolar. The scan rate is fixed to 100 mv/s.

The HOMO levels ($E_{HOMO}$) are calculated from the measured half wave potential of their first oxidation wave ($E_{1\ ox\ 1/2}$) using the following equation:

$$E_{HOMO} - (-4.8) = -[E_{1\ ox\ 1/2} - E_{ox\ 1/2}(Fc/Fc^+)]$$

wherein the ferrocene HOMO level value has been taken equal to -4.8 eV below the vacuum level according to Pommerehene and al. Adv. Mater. 7(6), 551-554 (1995) and wherein $E_{ox\ 1/2}(Fc/Fc^+)$ corresponds to the measured half wave potential of the ferrocene oxidation wave. For irreversible systems, $E_{pa\ 1}$ peak potential value of the first oxidation wave is used instead of the half wave potential $E_{1ox\ 1/2}$.

The LUMO levels ($E_{LUMO}$) are calculated from the measured half wave potential of their first reduction wave ($E_{1ox\ 1/2}$) using the following equation:

$$E_{LUMO} - (-4.8) = -[E_{1\ red\ 1/2} - E_{ox\ 1/2}(Fc/Fc^+)]$$

wherein the ferrocene HOMO level value has been taken equal to -4.8 eV below the vacuum level according to Pommerehene et al. Adv. Mater. 7(6), 551-554 (1995) and wherein $E_{ox\ 1/2}(Fc/Fc^+)$ corresponds to the measured half wave potential of the ferrocene oxidation wave. For irreversible systems, $E_{pc\ 1}$ peak potential value of the first reduction wave is used instead of the half wave potential $E_{1\ red\ ½}$.

The decomposition temperature was determined at the temperature where a weight loss of 1 % is observed upon heating a sample of the compound at a heating rate of 10 K/min.

## Claims

1. A process for the manufacture of dibenzosuberane compounds of formula (1)

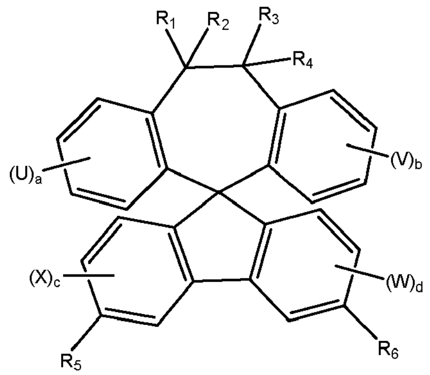

(1)

comprising the steps of

a) reacting a substituted aryl halide of formula (2) at a temperature in the range

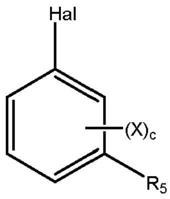

(2)

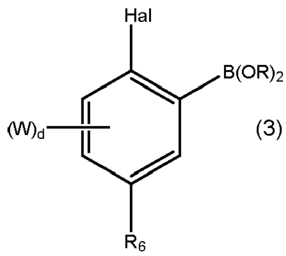

(3)

of from 60 to 200 °C with a boronic acid derivative of formula (3)
in the presence of a transition metal catalyst and a base to obtain a compound

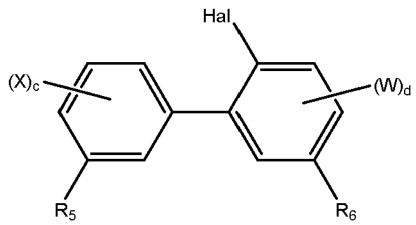

of formula (4)
b) subjecting the compound of formula (4) to a lithium-halogen exchange reaction at a temperature in the range of from -100 to 30°C to obtain lithiated biphenyl compound (5)

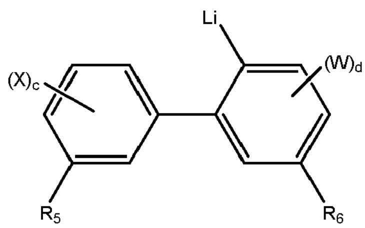

(5)

c) reacting the compound of formula (5) with a dibenzozosuberone compound of formula (6)

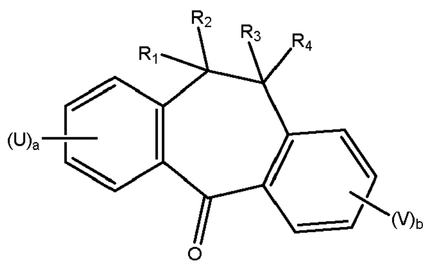

(6)

to obtain intermediate alcohol compound (7)

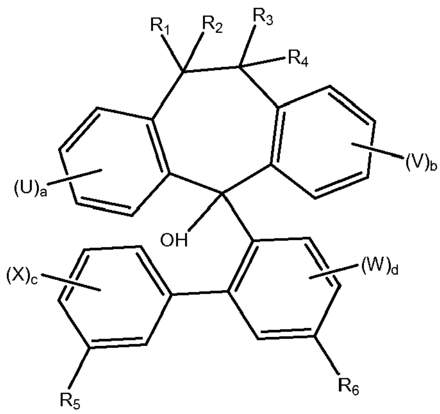

(7)

and thereafter

d) subjecting the intermediate compound (7) to a cyclisation reaction to obtain the compound of formula (1) wherein

Hal is a halogen atom,

R is hydrogen or a $C_1$-$C_8$ -alkyl group

$R_1$ to $R_4$, which may be the same or different, are selected from H or halogen, cyano, hydroxyl, alkyl, alkenyl, alkynyl, heteroalkyl, heteroalkenyl, heteroalkynyl and alkoxyl with 1 to 18 carbon atoms or $R_1$ and one of $R_3$ and $R_4$ form a chemical bond,

U, V, W, X , which may be the same or different at each occurrence, are halogen, cyano, hydroxyl, alkoxy, alkyl, alkenyl, alkynyl, heteroalkyl, heteroalkenyl, heteroalkynyl, aryl, heterocyclyl, and heteroaryl group,

$R_5$ and $R_6$, which may be the same or different at each occurrence, are hydrogen, halogen, cyano, hydroxyl, alkoxy, alkyl, alkenyl, alkynyl, heteroalkyl, heteroalkenyl, heteroalkynyl, aryl, heterocyclyl, and heteroaryl, provided that at least one of $R_5$ and $R_6$ is not hydrogen, and

a and b, which may be the same or different, are 0,1,2 or 3 and c and d, which may be the same or different, are 0, 1 or 2.

**2.** The process in accordance with claim 1 wherein the transition metal catalyst in step a) is a Pd catalyst.

**3.** The process in accordance with claim 1 or 2 wherein in step b) an alkyl lithium compound is used.

**4.** The process in accordance with any of claims 1 to 3 wherein step d) is carried out in the presence of a catalytic amount of a strong acid.

**5.** The process in accordance with any of claims 1 to 4, wherein in compound (2) $R_5$ is selected from the group consisting of hydrogen and

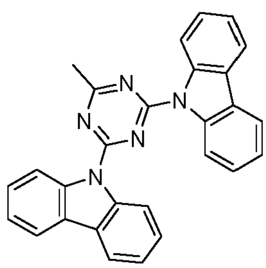

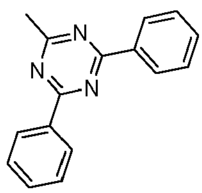

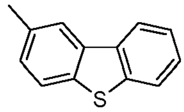

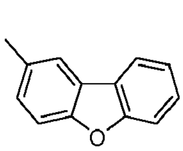

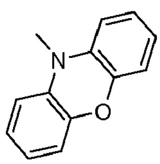

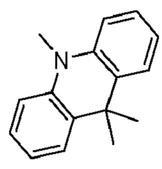

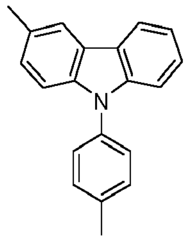
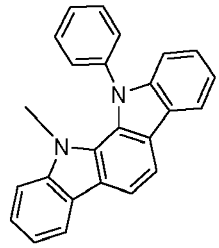
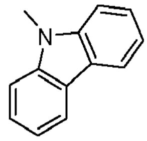

**6.** The process of any of claims 1 to 5, wherein in compound (3) $R_6$ is selected from the group consisting of hydrogen and

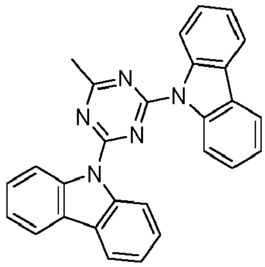
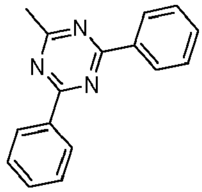
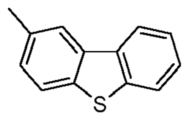

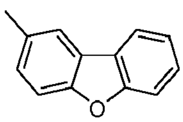
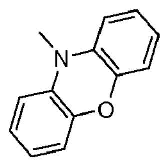
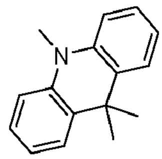

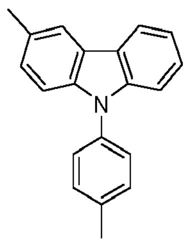
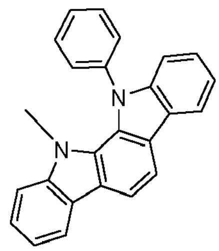
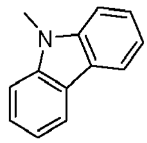

wherein the aromatic rings may be substituted by one or more substituent or substituents selected from the group consisting of halogen, hydroxy, alkoxy, alkyl, alkenyl or alkinyl, heteroalkyl, heteroalkenyl, heteroalkynyl, aryl, heterocyclyl, and heteroaryl.

**7.** The process in accordance with any of claims 1 to 6 wherein step a) is carried out in a solvent selected from the group consisting of toluene, xylene, tetrahydrofuran, dioxane, tetralin, quinolone, nitrobenzene, dimethyl sulfoxide and N,N-dimethylformamide.

**8.** Compounds of formula (8)

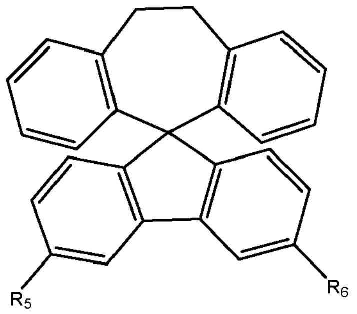

(8)

wherein $R_5$ and $R_6$, which may be the same or different at each occurrence, are hydrogen, halogen, cyano, hydroxyl, alkoxy, alkyl, alkenyl, alkynyl, heteroalkyl, heteroalkenyl, heteroalkynyl, aryl, heterocyclyl, and heteroaryl, provided that at least one of $R_5$ and $R_6$ is not hydrogen.

**9.** Compounds in accordance with claim 8 selected from the group consisting of

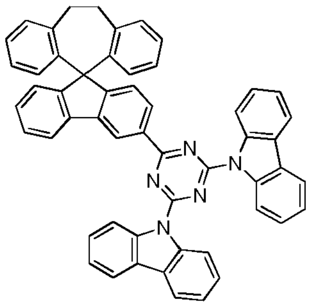

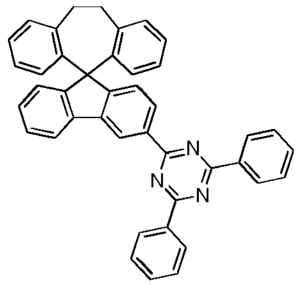

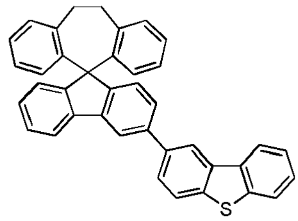

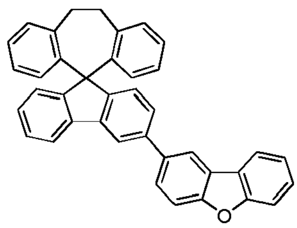

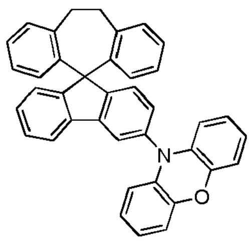

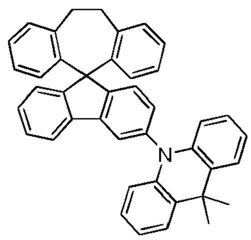

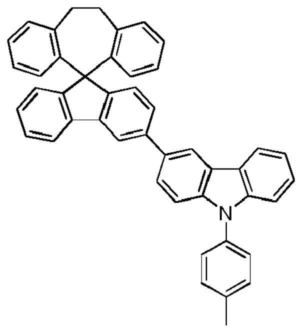

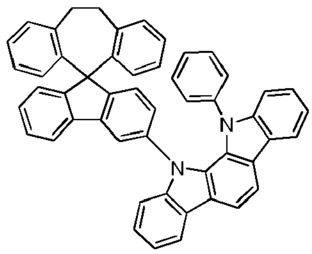

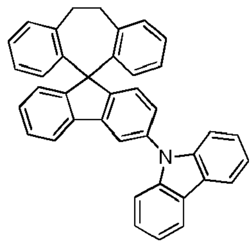

wherein the aromatic rings may be substituted by one or more substituent or substituents selected from the group consisting of halogen, hydroxy, alkoxy, alkyl, alkenyl or alkinyl, heteroalkyl, heteroalkenyl, heteroalkynyl, aryl, heterocyclyl, and heteroaryl.

Europäisches Patentamt
European Patent Office
Office européen des brevets

# EUROPEAN SEARCH REPORT

Application Number
EP 14 19 9899

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CHIEN-TIEN CHEN ET AL: "SUPPORTING INFORMATION: Doubly Ortho-Linked Quinoxaline/Diphenylfluorene Hybrids as Bipolar, Fluorescent Chameleons for Optoelectronic Applications", JOURNAL OF THE AMERICAN CHEMICAL SOCIETY, vol. 128, no. 34, 1 August 2006 (2006-08-01), pages 10992-10993, XP055167959, ISSN: 0002-7863, DOI: 10.1021/ja062660v<br>* page S3, paragraph Spiro-fluorene-dibenzobuberene *<br>----- | 1-7 | INV.<br>C07D209/86<br>H01L51/56 |
| Y | LEE CHIL WON ET AL: "Carbazole modified terphenyl based high triplet energy host materials for blue phosphorescent organic light-emitting diodes", DYES AND PIGMENTS, vol. 101, 2012, pages 150-155, XP028772077, ISSN: 0143-7208, DOI: 10.1016/J.DYEPIG.2013.10.003<br>* page 150, paragraph Experimental Section 2.1.1 - page 151 *<br>----- | 1-7 | TECHNICAL FIELDS SEARCHED (IPC)<br>C07D<br>H01L |
| A | DIETER HELLWINKEL ET AL: "Transannulare Hydridverschiebung versus Cyclokondensation bei 5-(2-Biphenylyl)-10,11-dihydro-5H-dibenzo[a,d]cyclohepten-5-ol-Derivaten", CHEMISCHE BERICHTE, vol. 122, no. 8, 1 August 1989 (1989-08-01), pages 1595-1597, XP055167556, ISSN: 0009-2940, DOI: 10.1002/cber.19891220832<br>* paragraph [ExperimentalSectionPoint1a] *<br>-----<br>-/-- | 1-9 | |

The present search report has been drawn up for all claims

1

| Place of search | Date of completion of the search | Examiner |
|---|---|---|
| Munich | 9 February 2015 | Sotoca Usina, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 19 9899

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | KR 2012 0047038 A (SFC CO LTD [KR]) 11 May 2012 (2012-05-11) * claims 1-8; compounds 187-189 * ----- | 8,9 | |
| X,D | JP 2010 024149 A (TOYO INK MFG CO) 4 February 2010 (2010-02-04) * claims 1-13; compounds 141-144, 149-150 * ----- | 8,9 | |

TECHNICAL FIELDS SEARCHED (IPC)

The present search report has been drawn up for all claims

1

| Place of search | Date of completion of the search | Examiner |
|---|---|---|
| Munich | 9 February 2015 | Sotoca Usina, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 19 9899

This annex lists the patent family members relating to the patent documents cited in the above-mentioned European search report. The members are as contained in the European Patent Office EDP file on The European Patent Office is in no way liable for these particulars which are merely given for the purpose of information.

09-02-2015

| Patent document cited in search report | Publication date | Patent family member(s) | Publication date |
|---|---|---|---|
| KR 20120047038 A | 11-05-2012 | NONE | |
| JP 2010024149 A | 04-02-2010 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 2012047038
- JP 2010024149 A


### Non-patent literature cited in the description


- *Chem. Rev.,* vol. 95 (7), 2457-2483
- *Chem. Soc. Rev.,* vol. 42 (12), 5270-5298
- **SU, S-J. ; CAI, C. ; KIDO, J.** *Chem. Mater.,* 2011, vol. 23, 274-284
- **LEE, C. W. ; IM, Y. ; SEO, J-A. ; LEE, J. Y.** *Chem. Commun.,* 2013, vol. 49, 9860-9862
- **LEE, C. W. ; LEE, J. Y.** *Dyes Pigments,* 2014, vol. 101, 150-155
- **POMMEREHENE.** *Adv. Mater.,* 1995, vol. 7 (6), 551-554
- **POMMEREHENE et al.** *Adv. Mater.,* 1995, vol. 7 (6), 551-554